# EUROPÄISCHE PATENTSCHRIFT

(11) **EP 0 974 643 B1**
(45) Veröffentlichungstag und Bekanntmachung des Hinweises auf die Patenterteilung: **01.10.2003**
(21) Anmeldenummer: 99890239.9
(22) Anmeldetag: 16.07.1999
(51) Int. Cl.: C12M 1/107

(54) **Verfahren und Vorrichtung zum biologischen anaeroben Abbau von organischen Abfällen unter Bildung von Biogas**
Process and system for the biological anaerobic digestion of organic waste with biogas production
Procédé et installation pour la dégradation biologique anaérobique de déchets organiques avec production de biogaz

(30) Priorität: 16.07.1998 AT 123498
(43) Veröffentlichungstag der Anmeldung: 26.01.2000
(73) Patentinhaber: Innovative Umwelttechnik Gesellschaft m.b.H, 2824 Seebenstein (AT)
(72) Erfinder: Rassaerts, Heinz, Dipl.-Ing. Dr.tech, 5020 Salzburg (AT)
(74) Vertreter: Itze, Peter, Dipl.-Ing.

(56) Entgegenhaltungen:
- EP-A- 0 033 017
- EP-A- 0 620 273
- EP-A- 0 647 605
- WO-A-96/02469
- CH-A- 628 007
- DE-A- 3 049 302
- DE-A- 3 105 081
- DE-A- 4 308 920
- DE-C- 243 020
- FR-A- 914 808
- US-A- 4 684 468
- US-A- 5 601 720

## Beschreibung

Die Erfindung bezieht sich auf ein Verfahren zum biologischen anaeroben Abbau von organischen Abfällen unter Bildung von Biogas, bei welchem frischer, gegebenenfalls mit in Fermentation befindlichem Material vermischter, Abfall mit einem hohen Trockensubstanzgehalt auf das in Fermentation befindliche Material aufgegeben wird.

Im Gegensatz zur Kompostierung, die seit langem betrieben wird, ist die anaerobe Fermentation (Vergärung, Faulung) von Abfällen ein vergleichsweise junger Zweig der Abfallwirtschaft. Die wesentlichen Ziele der anaeroben biologischen Behandlung sind:
- Reduzierung des Abfallvolumens
- Stabiliserung der organischen Abfälle
- Rückführung der organischen Substanz als Bodenverbesserer und zur Kompensation von Humusverlusten in den Boden
- Nutzung des Energiepotentials der leicht abbaubaren Fraktion für technische Zwecke.

Beim anaeroben Abbau von organischen Abfällen werden die organischen Kohlenstoffverbindungen in einem geschlossenen System unter Abwesenheit von Sauerstoff biochemisch zu Biogas, einer Mischung aus energiearmem Kohlendioxid (CO2) und energiereichem Methan (CH4) umgewandelt. Bis auf wenige Ausnahmen sind die meisten biogenen Stoffe unter anaeroben Bedingungen abbaubar. Der anaerobe Abbauprozeß findet nicht in einem Schritt statt, sondern verläuft unter Beteiligung verschiedener, voneinander abhängiger Mikroorganismengruppen in vier aufeinanderfolgenden Stufen:

| | |
|---|---|
| 1. Hydrolyse | |
| 2. Säurebildung | (acidogene Phase) |
| 3. Essigsäurebildung | (acetogene Phase) |
| 4. Methanbildung | (methanogene Phase). |

Bei der Fermentation ist die Erzielung hoher Raum - Zeit - Ausbeuten das zentrale reaktionstechnische Problem. Je komplexer das Stoffgemisch vom chemischen Aufbau her ist, wie dies im besonderen Maße bei Bioabfällen gegeben ist, umso schwieriger ist diese Forderung zu erfüllen. Aufgrund dieser besonderen stofflichen Komplexität der Bioabfälle sind selbst die Kinetik der wichtigsten Teilschritte nur unzureichend, wenn überhaupt, bekannt. Diese Informationen wären aber für eine optimale Ausführung des Verfahrens sowie für eine gesicherte Reaktorkonstruktion und Auslegung, Voraussetzung.

Da die biologischen Systeme auf Veränderungen bei der Stoffzufuhr nur träge reagieren, ist die Entwicklung einer "intelligenten" geregelten Reaktionsführung notwendig. Wie bereits oben ausgeführt, läuft der anaerobe Abbau organischen Materials über mehrere Zwischenstufen. Neben dem breiten Spektrum und den unterschiedlichen Generationszeiten der beteiligten Mikroorganismen sowie den komplizierten Wechselwirkungen zwischen fermentativer Säurebildung und

Methanogenese ist hier noch die von der chemischen Thermodynamik diktierte, regulative Rolle des Wasserstoffs auf die Essigsäurebildung durch die acetogenen Bakterien zu berücksichtigen. Wird dieser nämlich nicht hinreichend schnell von den Methanbakterien verwertet, so kommt es zu einer Akkumulation von Butter- und insbesondere Propionsäure im Reaktor und bei höheren Konzentrationen zu einer nachhaltigen Hemmung der Methanbildung, was zur starken Herabsetzung der Raum - Zeit - Ausbeute führt.

Vorliegend werden unter biologisch abbaubaren organischen Abfällen Küchenund Speiseabfälle mit keinen oder sehr geringen Mengen an biologisch nicht abbaubaren Fremdstoffen, wie sie durch spezielle getrennte Sammlung in den Haushalten erhalten werden (sogenannter Bioabfall), biologisch leicht abbaubare Schlämme und Suspensionen aus dem kommunalen und industriellen Bereich oder Bioabfälle aus den lebensmittelverarbeitenden Industrie- und Gewerbebetrieben etc., z.B. Brauereien, Brennereien, Konservenfabriken, Stärkefabriken, Zuckerfabriken bzw. aus allen anderen Bereichen wirtschaftlicher Tätigkeit, z.B. Großmärkte, Kantinen, Gaststätten, Fast Food Restaurants, Schlachthöfen, Antibiotikaerzeugung, Pflanzenproduktion verstanden.

Entsprechend bekannten Verfahren für den anaeroben Abbau von Haushaltsund Bioabfällen wird für die einstufige Verarbeitung Wasser hinzugefügt, bis Pulpen mit 10 - 15 % Feststoffgehalt erhalten werden, die anschließend fermentiert werden. Im kontinuierlichen Betrieb stellen sich im Fermenter Feststoffkonzentrationen von 8 - 12 % bezogen auf Masse ein, die im allgemeinen bei Verweilzeiten von 10 - 25 Tagen und bei Temperaturen von 35 - 55°C anaerob abgebaut werden. Der Inhalt der Fermenter wird dabei so in Bewegung gehalten, daß die frische Bioabfallpulpe mit der im Fermenter befindlichen anaeroben Biomasse vollständig durchmischt wird, um inaktive Zonen zu vermeiden. Unter solchen Reaktionsbedingungen läuft der mehrstufige anaerobe Abbaumechanismus nicht optimiert ab. Deshalb sind zweistufige anaerobe Fermentations-Verfahren entwickelt worden, bei welchen die saure Abbauphase und die basische Methanbildungsphase getrennt ablaufen. Bei solchen Verfahren sind große Wasservolumen im Kreise zu fahren, es fällt naturgemäß mehr Abwasser an, einige Verfahren benötigen zusätzlich Chemikalien, z.B. Natronlauge, teurere Apparate aus korrosionsbeständigen Werkstoffen sind erforderlich und letztlich sind komplexere Steuerungs- und Regelungseinrichtungen notwendig. In der Praxis hat sich gezeigt, daß damit wegen des komplizierten Verfahrens keine höheren Ausbeuten an Biogas erzielt werden.

Mit dem höheren Wassergehalt sinkt aber grundsätzlich die Raum - Zeit - Ausbeute, die für die Größe, Ausstattung und dementsprechend die Investitionskosten der Anlagen maßgebend ist. So werden bei den sogenannten einstufigen nassen Fermentations-Verfahren mit einem Reaktorzulauf von beispielsweise 10 % OTM (Organische-Trocken-Masse) eine im Vergleich geringe Raumbelastung Br von ca. 5 kg OTM/m³.d und Biogasproduktivität von ca. 3,0 m³ Biogas pro m³ Reaktorvolumen und pro Tag erzielt.

Zweistufige "nasse" Verfahren arbeiten bei niedrigen Feststoffkonzentrationen in der Methanisierungsstufe und verwenden Schlammbett-Reaktoren oder andere Reaktortypen aus der Abwassertechnik. Bei diesen Verfahren wird der Bioabfall zunächst zerkleinert und in Stofflösern die Fremdstoffe abgetrennt. Anschließend werden die Abfälle in Hydrolysereaktoren soweit wie möglich in Lösung gebracht und der nicht hydrolysierbare Restanteil durch Dekanter oder sonstige Dehydratisierungs-Apparate abgetrennt. Die erhaltene Flüssigphase, die maximal 3 % leicht abbaubaren Feststoff enthält, wird in der Folge in einem Festbett-Methanreaktor im Up-flow-Modus oder ähnlichen Reaktoren vergoren. Solche Verfahren sind technisch aufwendig, mit hohem Abwasseranfall und erheblichem Anfall an Abfällen sowie mit hoher Prozeßenergie verbunden, welche den vermarktbaren Energieüberschuß reduziert.

Es ist auch bereits bekannt, Verfahren mit höherem Trockensubstanzgehalt arbeiten zu lassen, jedoch führten in der Vergangenheit zäh pastöse Medien bei Mischvorgängen, Stoff- und Energieaustauschprozessen oft zu schweren Verfahrensstörungen. Es wurden daher bisher bevorzugt Naßfermentationsverfahren konzipiert und ausgeführt, obwohl bekannt war, daß sich bei trockenen Verfahren geringere Reaktionsvolumina ergeben und eine wesentlich geringere Förderung von Stoffströmen erforderlich ist.

US-A-4,684,468 offenbart ein Verfahren zum biologischen anaeroben Abbau von organischen Abfällen, bei dem ein Teil des in Fermentation befindlichen Materials, nach einer Verweilzeit, aus dem Bioreaktor abgeführt, mit frischem Material vermischt und zurück in den Bioreaktor in eine Materialstromschlaufe eingebracht wird. Der gesamte Inhalt des Bioreaktors ist als Methanbildungsbereich anzusehen.

Der Erfindung liegt die Aufgabe zugrunde, ein Verfahren der eingangs genannten Art zu schaffen, bei welchem trotz hohen Trockensubstanzgehaltes die angeführten Nachteile nicht auftreten.

Erfindungsgemäß wird diese Aufgabe dadurch gelöst, daß vorzugsweise nach Beendigung der Zugabe frischen, gegebenenfalls mit in Fermentation befindlichem Material vermischten Materials, das in Fermentation befindliche Material, gegebenenfalls nach Entwässerung, im Kreislauf geführt wird, wobei das im Kreislauf rückgeführte Material in den Bereich der Methanbildung, etwa in halber Höhe der Substratschicht, eingebracht wird. Es wird also das Fermentationsmaterial in zwei Stoffströme getrennt, wobei der erste größere mit Frischmaterial zwecks Impfung vermischt und oben im Reaktor aufgegeben wird, wobei der zweite, kleinere, zwecks Nachimpfung etwa in der Mitte des Reaktors ohne Frischmaterial aufgegeben wird. Die derartig im Kreis geführte Reaktionsmasse beimpft somit die im Bereich der Methanbildung befindliche Masse immer neu, sodaß in diesem Bereich immer eine sehr hohe Bakterienpopulation herrscht. Außerdem werden durch das Im-Kreislauf-Führen der Reaktionsmasse Entmischungen oder Verkrustungen vermieden, womit ebenfalls eine konstant hohe Reaktionsgeschwindigkeit eingehalten werden kann.

Bei der Trockenfermentation ist die vollständige Reaktordurchmischung die Operation, die den erforderlichen Stoffübergang und Abtransport des Biogases bestimmt. Ist die Feststoffkonzentration so hoch, daß sich eine plastische Reaktionsmasse bildet, kann unter Umständen eine ausreichende Versorgung der Bakterien mit abbaufähigem Substrat nicht mehr gegeben sein. Dementsprechend bildet bei diesen Verfahren die Vermeidung von Temperatur- und Stoffgradienten sowie Hemmungen des Biogasaustrages das technische Problem, dem nur durch vergleichsweise mäßige Raum-Zeit-Ausbeuten begegnet werden kann.

Vorteilhafterweise kann frischer Abfall mit in Fermentation befindlichen Materialien im Verhältnis von 1: 3 bis 1: 8, insbesondere 1: 4 bis 1 : 6, vermischt werden. Damit wird das saure Frischmaterial rasch auf einen für die Methanisierung günstigen pH-Wert gebracht. Als besonders günstig für die Bakterienpopulation hat sich herausgestellt, wenn zur Nachimpfung in Fermentation befindliches Material im Kreislauf geführt wird, u.zw. bezogen auf frischen Abfall im Verhältnis von 4 : 1 bis 1 : 1, insbesondere 3 : 1 bis 2 : 1. Für einen konstanten Verfahrensablauf kann maximal die Hälfte, vorzugsweise 1/3 bis 1/4 des gesamten in Fermentation befindlichen Materials im Kreislauf geführt werden.

Um einen für die Methanisierung günstigen Abbaugrad des Materials zu erhalten, kann der pH-Wert des frischen Abfalls zwischen 3 und 5 eingestellt werden. Damit werden besonders günstige Bedingungen für die Hydrolyse, die Säurebildung und die Essigsäurebildung erzielt.

Für ein besonders ausgewogenes Verhältnis zwischen Reaktionsgeschwindigkeit, Reaktionsvolumen und Durchmischung des Substrates kann die Feststoffkonzentration des für das Umwälzen abgezogenen, in Fermentation befindlichen Materials zwischen 20 und 50 Gew.-%, vorzugsweise zwischen 24 und 40 Gew.-% liegen. Dabei kann die Feststoffkonzentration durch Entwässerung eines Teiles des umgewälzten Gutes und Wiedervereinigen des entwässerten Gutes mit dem Rest des umgewälzten Gutes eingestellt werden. Zum Einstellen der Feststoffkonzentration kann dem umgewälzten Gut Wasser, Abwasser, Dünnschlamm oder Gülle zugesetzt werden, wodurch auch diese Substanzen dem Abbau zugeführt und gleichzeitig zur Steuerung des Verfahrensablaufes eingesetzt werden. Weiters kann zum Entfernen von Schadstoffen das in Fermentation befindliche Material von der Hauptmenge abgezogen, entwässert, vorzugsweise gepreßt, und die abgeführte Flüssigkeitsmenge durch Schadstoffe anderer Flüssigkeiten, z.B. Wasser, Abwasser, Dünnschlämme oder Gülle, vor dem Rückführen in die Hauptmenge ersetzt werden. Dies ermöglicht es, Schadstoffe, die sich in der flüssigen Phase des Reaktionsgutes ansammeln, aus dem Reaktionsgut auszubringen, womit eine Inhibierung des Verfahrensablaufes durch derartige giftige Schadstoffe vermieden wird. Schließlich kann der Ammoniumstickstoff in dem in Fermentation befindlichen Gut auf maximal 4 g/kg gehalten werden, wobei gegebenenfalls dem Gut Nährstoffe für die anaeroben Mikroorganismen beigemengt werden. Dies vermindert indirekt überschüssige Stickstoffverbindungen im Biogas.

Die durch das erfindungsgemäße Verfahren erzielten Vorteile werden nachstehend anhand des Verfahrens nocheinmal zusammengefaßt.

Die gegenständliche Erfindung ergibt nun ein neues, sehr effektives Verfahren zum anaeroben Abbau von biologisch abbaubaren organischen Abfällen mit hoher Raum - Zeit - Ausbeute, wie insbesondere von der organischen Fraktion des Hausabfalls, d.h. von Bioabfall, von industriellen organischen abbaubaren festen Abfällen und Schlämmen sowie sonstigen organischen Abfällen unterschiedlicher Herkunft, sofern diese leicht hydrolysierbar und anaerob abbaubar sind. Zu diesem Zweck werden die Abfälle mit einem Trockenmassegehalt (im folgenden "TM" abgekürzt) von 20 - 35 % am Kopf des Reaktors aufgegeben, welche im stationären Betrieb bei Anwendung der erfindungsgemäßen Vorrichtung im Reaktor eine Suspension mit einem Konzentrationsgradienten an Feststoffen bildet. Die gegenüber der Reaktionsmasse spezifisch leichteren festen organischen Bestandteile sammeln sich im verstärkten Maße im oberen Teil des Reaktors an und werden dort unter mikrobiell vorteilhafteren Bedingungen hydrolysiert, als sie im unteren Teil des Reaktors herrschen. Im oberen Bereich des Reaktors stellt sich ein pH-Wert von ca. 7 ein, im unteren Bereich ergeben sich bei einem pH-Wert von 8,0 bis 8,3 Bedingungen, die die Methanbildung begünstigen.

Bei TM-Konzentrationen zwischen 20 und 33 % ist die Suspension im Reaktor bei erfindungsgemäßer Ausführung des Verfahrens so stabil, daß keine störende Entmischung auftritt. Vorteilhafterweise wird die Fermentation bei TM-Konzentrationen von 24 - 30 % ausgeführt. Die schnelle Abbaubarkeit und der hohe Wassergehalt der Bioabfälle führt zu einer "Verflüssigung" der Reaktionsmasse im Reaktor erfahrungsgemäß um ca. 5 % TM. Das anfangs stichfeste Fermentationssubstrat wird wässriger. Unter 20 % TM in der Reaktionsmasse beginnt der Feststoffgradient im Reaktor sich nachteilig auf das Reaktionsverhalten auszuwirken, da die Methanbakteriendichte, die vorwiegend an die Feststoffpartikel des Substrates gebunden ist, stark abnimmt. Dies führt zu einer Abbauminderung bis zur Abbauhemmung und damit zum Verlust an Raumbelastung des Reaktors.

Es ist nun ein wesentliches Merkmal der Erfindung, daß entwässertes fermentiertes Substrat an geeigneter Stelle, die sich in der unteren Volumenshälfte des Reaktors vorzugsweise zwischen 30 - 50 % des Reaktorvolumens befindet, eingetragen wird, um die Feststoffkonzentration und Methanbakteriendichte in diesem Abbaubereich zu erhöhen. Auch der umgekehrte Fall bei zu hoher Feststoffkonzentration wäre möglich, indem Preßwasser aus der Entwässerungsvorrichtung zurück in den Reaktor geführt wird, um optimale Reaktionsbedingungen aufrechtzuhalten. Die erfindungsgemäße Vorrichtung und Reaktionsführung erlaubt somit die laufende optimale Einstellung der Reaktions- und Abbaubedingungen in Relation zum Bioabfall - Input. Damit sind im kontinuierlichen Betrieb gegenüber vergleichbaren bekannten Verfahren höhere Raum - Zeit - Ausbeuten erreichbar. Aufgrund der so erzielbaren Stabilität der Suspension können ohne spezielle Maßnahmen die erfindungsgemäß erforderlichen 25 - 45 % Reaktormasse entnommen werden, die zum größeren Teil als Impfgut mit Frischmaterial vermischt über die Rohrschlaufe am Kopf des Reaktors wieder aufgegeben werden. Ein kleinerer Teil davon wird in Abhängigkeit von den Eigenschaften des frischen Bioabfalls zur "sekundären Impfung" in den Reaktor zurückgeführt.

Ein wesentlicher Vorteil des Verfahrens ist darin gegeben, daß die leicht abbaubaren Bioabfälle mit TM von 25 - 35 % direkt, allenfalls nach einer mechanischen Vorbehandlung durch Zerkleinerung und Absiebung von flächenhaften Fremdstoffen, wie z.B. Plastikfolien, über die Mischeinrichtung in den Reaktor aufgegeben werden können.

Gegenüber den herkömmlichen Verfahrensweisen ergeben sich noch folgende Vorteile:

Bei einstufigen trockenen Verfahren wird eine TM-Konzentration zwischen 25 - 40 %, vorzugsweise 30 - 35%, angestrebt. Wegen des hohen Wassergehaltes im Bioabfall können im stationären kontinuierlichen bzw. halbkontinuierlichen Betrieb Papierabfälle oder andere trockene Abfälle zugemischt werden oder der Wasserüberschuß durch Entwässerung eines großen Teils des Reaktorinhaltes entfernt werden.

Das erfindungsgemäße Verfahren unterscheidet sich auch wesentlich und vorteilhaft von nassen zweistufigen Verfahren in der Weise, daß durch die Gradienten der Stoffkonzentrationen und des pH-Wertes in der Reaktionssuspension die Hydrolyse und Methanbildung in einem einzelnen Reaktor unter den begünstigenden Bedingungen ablaufen, wie sie im Zweistufen-Verfahren geplant in getrennten Apparaten für Hydrolyse und Methanisierung, durchgeführt werden. In diesen Fällen sind jedoch für den Stofflöser und Hydrolysereaktor große Mengen an Prozeßwasser zu rezirkulieren und Feststoffe zu dehydratisieren.

Bei einer erfindungsgemäßen Vorrichtung zur Durchführung des Verfahrens, bei welcher ein Reaktionsgefäß mit Abzugsleitungen für Biogas, eine Beschickungseinrichtung, eine Austrageeinrichtung, eine Entwässerungsvorrichtung und eine Mischvorrichtung vorgesehen sind, ist erfindungsgemäß die Beschickungseinrichtung als Beschickungsschlaufe ausgebildet, die oberhalb der Substratschicht in das Reaktionsgefäß einmündet und zusätzlich eine Umwälzschlaufe vorgesehen, die im Bereich von 25 bis 70 %, insbesondere 30 - 50 %, der Substratschichthöhe in das Reaktionsgefäß einmündet. Damit wird erreicht, daß das rückgeführte Material in jenen Bereich des Reaktionsgefäßes eingebracht wird, in welchem der Bereich der Methanbildungsphase ist.

Bevorzugterweise kann sowohl die Beschickungsschlaufe als auch die Umwälzschlaufe von der Mischvorrichtung ausgehen, was die Möglichkeit ergibt, daß nach Bedarf sowohl bei der Neubefüllung der Anlage als auch im Umwälzbetrieb zu dem umgewälzten Gut eventuell frisches, zu vergärendes Gut oder aber auch Preßwasser u.dgl., zugesetzt werden kann. Dabei kann das Verhältnis von Durchmesser zur Höhe des Reaktionsgefäßes zwischen 1 : 1,3 bis 1 : 4 betragen, was den Vorteil hat, daß innerhalb des Reaktionsgefäßes keine vollständige Rückmischung auch bei niederem Trockenmassegehalt vor sich geht. Um tote Zonen beim Austragen des Gutes aus dem Reaktionsgefäß zu vermeiden, kann die Austrageeinrichtung durch eine oder mehrere Förderschnecken gebildet sein, wobei der Boden des Reaktionsgefäßes zu der bzw. den Förderschnecken hin geneigt verläuft.

Weitere vorteilhafte Details werden anhand der nachfolgenden Zeichnung näher beschrieben, welche ein Ausführungsbeispiel der erfindungsgemäßen Vorrichtung wiedergibt, ohne dabei in irgendeiner Weise den Schutzbereich der angeschlossenen Patentansprüche zu beschränken.

Die dargestellte Vorrichtung besteht aus einem geschlossenen Reaktor 1, der Beschickungsschlaufe 2 zur Beschickung des Reaktors, der Rückführungsschlaufe 32 zur Substratrückführung, der Austragsvorrichtung 5 zum Austrag der Reaktionsmasse, der Entwässerungsvorrichtung 19 zum Dehydratisieren der Reaktionsmasse und der Mischvorrichtung 6 zum Mischen und Impfen der frischen organischen Abfallmasse 24 mit der Reaktionsmasse 10. Die Beschickungsschlaufe besteht aus der Verdrängerpumpe 7, der Förderleitung 8 samt Wärmetauscher 9 und der aus einem oder mehreren Rohren 3 gebildeten Beschickung des Reaktors 1 mit den automatischen Absperrschiebern 4. Die Rückführungsschlaufe, über welche Reaktionsmasse, optional entwässert, in den Reaktor rückgeführt wird, setzt sich aus dem Förderband 33, der Rohrleitung 34 und den automatischen Absperrschiebern 35, 36 und 37 zusammen. Die Austragsvorrichtung besteht aus zwei im Hosenboden des Reaktors 1 montierten Förderschnecken 11, aus dem Zwischenbehälter 13 mit den automatischen Absperrschiebern 12 und 14 in den zuführenden und abführenden Rohrstücken. Der Zwischenbehälter 13 ist über die Förderschnecke 15 und den automatischen Absperrschieber 27 mit dem Mischer 6 und über die Förderschnecke 16 sowie das Rohrstück mit Absperrschieber 20 mit der Mischvorrichtung für den Eintrag eines Flockungsmittels 21 verbunden.

Die Entwässerungsvorrichtung enthält als Kernstück die Schneckenpresse 19, optional Kolbenpresse oder Dekanter, die über den Mischbehälter 18 und Rohrstück mit Absperrschieber 22 beschickt wird. Das Preßwasser gelangt via Auslaß 29 in einen Lagertank, der erforderlichenfalls als Zwischenstufe für die weitere Behandlung des Preßwassers oder als Vorlagebehälter für die Rückführungsvorrichtung via Mischer 6 dient. Der Preßkuchen, d.h. die entwässerte Reaktionsmasse, wird via Auslaß 30 ausgetragen und mittels Förderband 31 zur Nachbehandlung, vorzugsweise Kompostierung, transportiert.

Die Mischvorrichtung besteht aus dem Mischer 6, der Förderleitung 25 mit automatischem Absperrschieber 26, der verbunden ist mit Verdrängerpumpe 7, dem Förderband 24, mit dem der frische organische Abfall in den Mischer eingebracht wird, und den zusätzlichen Rohranschlüssen 23 und 39 für den optionalen Eintrag von Dünnschlämmen und von Dampf zur optionalen Erwärmung des Reaktionsgemisches.

Am Kopf des Reaktors 1 wird über das Entgasungsrohr 28 das gebildete Biogas abgezogen und in den Biogastank gefördert. Das erzeugte Biogas dient zur Gewinnung von elektrischer Energie in Biogasmotoren, deren im Kreislauf geführtes Kühlwasser von 85 - 90°C zur Erwärmung des Reaktorinputs im Wärmetauscher 9 verwendet wird.

Zur Vermeidung, gegebenenfalls zum Aufbrechen einer dichten Schwimmdecke im Reaktor 1, welche die reibungslose Entgasung der Reaktionsmasse 10 behindert, kann verdichtetes Biogas über die Rohrleitung 38 eingepreßt werden.

Die oben beschriebene Vorrichtung wird in der folgenden Weise verwendet und betrieben:

Zur Inbetriebnahme der gesamten Fermentationsanlage wird der Reaktor 1 zunächst über die Beschickungsschlaufe 2 mit Impfgut (Inoculum) befüllt. In der Startphase des Reaktors wird fremdes Impfgut benötigt, um so schnell wie möglich den stationären Betriebszustand ohne mikrobioelle Fehlreaktionen zu erreichen, die ohne Impfung unter Umständen mehrere Monate dauern kann. Mit entsprechender Impfung wird die einwandfreie Fermentation schon nach vier Wochen erreicht. Ein solches Impfgut kann naturgemäß in vorteilhafter Weise einem im laufenden Betrieb stehenden Reaktor entnommen werden, in welchem die Bioabfälle etwa trocken unter thermophilen Bedingungen fermentiert werden. Es kann ebenso entwässertes Reaktionsgut aus den Faulbehältern kommunaler Kläranlagen oder aus gut funktionierenden nassen Fermentern für Bioabfälle, organische Klärschlämme, Gülle und sonstige biologisch abbaubare organische Materialien verwendet werden. Das Impfgut soll den gewöhnlichen Besatz an anaeroben methanbildenden Bakterien-Populationen, beispielsweise der Spezies Methanosarcina, Methanococcus, Methanothrix, etc. enthalten. Der TM-Gehalt des Impfguts sollte sich aus Gründen der einfacheren Handhabung zwischen 20 - 35 % TM bewegen, es können aber auch solche bis 15 % TM herab verwendet werden. In der Regel setzt man wengistens eine dem Drittel des Reaktorvolumens entsprechende Menge an Impfmasse ein, welche über den Wärmetauscher 9 oder optional durch direkte Dampfeinspeisung 39 bei thermophiler Fahrweise auf 50 - 60, vorzugsweise 55°C, aufgewärmt wird. Auch eine Fahrweise bei mesophilen Temperaturen ist möglich, doch ist wegen der langsameren Abbaukinetik die Raum - Zeit - Ausbeute herabgesetzt. Nachdem die gesamte Impfmasse auf Temperatur gebracht ist, wird über die Austragsvorrichtung 5 und Förderschnecke 15 das Impfgut mit dem über das Förderband 24 eingebrachten Frischgut mit maximaler Korngröße von 50 mm im Mischer 6 vermischt. Das Mischungsverhältnis Impfgut zu Frischmaterial sollte nicht weniger als 3 : 1 und maximal 1 : 8 betragen. Über die Reaktorschlaufe aus der Verdrängerpumpe 7, Förderrohr 8, Wärmetauscher 9, Aufgabevorrichtung 2 mit Verteiler 3 wird der Vorgang solange wiederholt, bis der Reaktor gefüllt ist und den stationären Betriebszustand erreicht hat.

Durch die hoch aktiven Bakterien-Populationen im Impfgut stellt sich ein pH-Wert und Konzentrationsgefälle im Reaktor ein, das praktisch nur durch den TM-Gehalt und den Aufgabezustand des Bioabfalls bestimmt wird. Im Normalfall sind die Bioabfälle insbesondere bei wärmeren Außentemperaturen durch die notwendige Aufenthaltszeit in den Sammelbehältern in den beginnenden Abbau der organischen Bestandteile übergegangen. Im langzeitigen Mittel wurden pH-Werte zwischen 4 - 5 gemessen. Man kann den frischen Bioabfall, sofern er noch nicht diesen Säuregrad aufweist, zwischenlagern, bis er diesen pH-Wert aufweist. Dies ist in der Regel unter kontrollierten Lagerungsbedingungen und Temperaturen > 15°C nach 2 - 3 Tagen gegeben. Der saure Bioabfall, der je nach Qualität mechanisch mehr oder weniger aufbereitet ist, wird mit einer Korngröße von max. 50 mm mittels Förderband 24 im Mischer 6 mit der über die Förderschnecke 15 ausgetragenen Reaktionsmasse als Impfgut vermischt. Die Reaktionsmasse weist abhängig von den chemischen Bestandteilen des Bioabfalls bzw. der organisch abbaubaren Abfälle einen basischen pH-Wert Bereich von ca. 8,0 - 8,3 auf. Durch die Vermischung des sauren Frischmaterials mit der basischen Reaktionsmasse, im Effekt Impfgut, treten chemische Reaktionen auf, die den pH-Wert herabsetzen und das Milieu für die Vorstufen der Methanisierung begünstigen. Das Mischungsverhältnis von Frischmasse zu Reaktionsmasse beträgt vorzugsweise 1 : 4 bis 1 : 6. Höhere Mischungsverhältnisse sind reaktionstechnisch möglich, doch setzen sie den Wirkungsgrad des Reaktors herab.

Durch die so eingestellten hochaktiven Reaktionsbedingungen findet der anaerobe Abbau eines großen Teils der organischen Masse in kurzer Zeit unter vehementer Biogasentwcklung und unter Zunahme des Wassergehaltes statt. Optimale Verhältnisse herrschen, wenn der TM-Gehalt in der Reaktionsmasse 10 innerhalb der Grenzen 25 - 30 % (= 70 - 75 % Wasser) liegt. Falls der TM-Gehalt erheblich unter 25 % absinkt, wird fermentierter Preßkuchen über den Mischer 6 und die Rückführungsschlaufe 32 in den Reaktor gefördert, um den TM-Gehalt anzuheben und im Effekt die Stoffwechselaktivität der Methanisierung zu beschleunigen.

Die ergänzende Beimpfung durch Kreisführung der Reaktionsmasse über die durch 32 gebildete Reaktionsschlaufe bildet unabhängig von der Notwendigkeit einer Aufkonzentrierung einen wesentlichen Bestandteil des täglichen Reaktionsablaufes. Das Masseverhältnis dieses Kreislaufes beträgt bezogen auf die Frischmasse 1:1 bis 1:4, vorzugsweise 1:2 bis 1:3.

Im Tagesablauf wird der Reaktor während max. 8 h mit frischen Bioabfällen beschickt. Zu gleicher Zeit wird über die Austragsvorrichtung 5 und der Entwässerungsvorrichtung 19 die fermentierte Reaktionsmasse ausgetragen und auf 50% TM, wenn erforderlich mit Flockungsmittel, entwässert. Das Preßwasser gelangt zur weiteren Behandlung in den Preßwassertank, der Preßkuchen wird kompostiert.

Nach Beendigung der täglichen Beschickungsphase tritt die Phase der Nachbeimpfung ein. Zu diesem Zweck werden die Absperrschieber 36 und 37, die in die Beschickungsphase geschlossen sind, geöffnet und der Absperrschieber 35 geschlossen. Die Reaktionsmasse 10 nimmt nun den Weg über Förderschnecke 15, Mischer 6, Verdrängerpumpe 7, Wärmetauscher 9 und Eintragsrohr 34 in den Reaktor. Im Fall der Aufkonzentrierung des Feststoffgehaltes von 10 wird Preßkuchen aus 19 mit dem Förderband 33 in den Mischer eingetragen und dort mit der nachimpfenden Reaktionsmasse vermischt in den Reaktor gefördert. Die Reaktionsmasse bleibt anschließend in der Regel 10 - 12 h über Nacht sich selbst überlassen, wobei sich die entsprechenden Konzentrationsschwerpunkte ausbilden. Das während jeder Phase gebildete Biogas wird über das Gasrohr 28 durch eine Verdichterstation abgesaugt und z.B. in einem Gasbehälter gesammelt. Zur Zerstörung einer sich allenfalls bildenden Schwimmdecke kann durch die Gasleitung 38 verdichtetes Biogas eingeblasen werden, wobei auf die Vermeidung von stärkerer Schaumbildung geachtet werden muß. Nach der Ruhephase beginnt erneut die Beschickungsphase, in der die zuzuführende Frischmasse der ausgetragenen Reaktionsmasse und der abgesaugten Biogasmasse entspricht. Die im Hosenboden angeordneten Austragsschnecken 11 fördern den Austrag in den Zwischenbehälter 13, der neben der Verteil- und Pufferfunktion auch als Schleuse wirken kann. Wenn die Reaktionsmasse unter 20% TM sinkt, wird zur Stabilisierung der Förderwirkung der Massenstrom über die Absperrschieber 12 und 14 geregelt.

Bei hohem Stickstoffgehalt in den frischen abbaubaren organischen Abfällen bzw. bei sehr starkem Abbau in der Reaktionsmasse, sind vorzugsweise Substrate mit hohem Kohlenstoffanteil wie etwa Papierabfälle, Holz- und Rindenabfälle, Preßkuchen aus Abwasserreinigungen (Papiererzeugung) oder auch durch aerobe Behandlung nitrifizierte - denitrifizierte Preßkuchen in den Reaktor 1 einzubringen, um den Gehalt an Ammoniumstickstoff nicht über 4g/kg Reaktionsmasse 10 ansteigen zu lassen.

Das beschriebene Verfahren erlaubt durch die erfindungsgemäße Konstruktion eines Doppelschlaufen - Reaktionssystems die unmittelbare Verwendung jeglicher abbaubarer organischer Abfälle, welche außer einer mechanischen Vorbehandlung durch Zerkleinern und Sieben keiner weiteren Behandlung durch Auflösen, Entwässern, Hydrolisieren, etc. bedarf. Es muß den Abfällen weder Wasser zugeführt noch entzogen werden.

Der Bereich des TM-Gehaltes der verarbeitbaren abbaubaren organischen Abfälle reicht von 2% TM (z.B. kommunale Abwässer) bis 70% TM (z.B. Holzabfälle). Trotzdem werden mit der erfindungsgemäßen Verfahrensweise und Vorrichtung hohe Raum - Zeit - Ausbeuten erzielt, die über dem bekannten technischen Verfahren liegen. Es werden Raumbelastungen Br von 7 - 11 kg OTM (organische Trockenmasse) pro m³ Reaktorvolumen und Tag gefahren und Biogas-Produktivitäten von 5 - 7 m³ pro m³ Reaktorvolumen und Tag erzielt. Das Reaktorvolumen kann dadurch vergleichsweise klein gehalten werden. Das Verhältnis der Massenströme zwischen der Beschickungsschlaufe und der Rückführungsschlaufe beträgt dafür vorzugsweise 2 bis 4, kann aber im Bereich von 1 bis 10 liegen.

Die erfmdungsgemäße Verfahrensweise wird durch die folgenden Beispiele deutlich gemacht:

### Beispiel 1:

In einen Reaktor mit 22m³ Inhalt der vorbeschriebenen Konstruktion mit D:H = 1:2 wurden in täglichen Chargen 7000 kg/Woche getrennt gesammelter Bioabfall mit 30% TM aufgegeben. Der TOC-Gehalt betrug 38% bezogen auf TM und war zu 90% biologisch abbaubar. Das Beschickungsverhältnis Frischmaterial zu Reaktionsmasse war 1:5, in der Rückführungsschlaufe wurde mehr als die doppelte Masse im Bezug auf Frischmaterial umgewälzt. Die Reaktionstemperatur betrug 55°C und wurde durch Erwärmung des Beschickungsgemisches in einen Platten-Wärmetauscher erreicht. In einer Woche wurden 1250 kg Biogas bei einer hydraulischen Verweilzeit von 18 Tagen produziert. 6500 kg an Reaktionsmasse wurden in der Woche ausgetragen und auf 50% TM entwässert. Davon wurden 15 % im entwässerten Zustand wieder in den Reaktor via Eintragsmischer zurückgeführt. Auf diese Weise konnte ein erhöhter TM-Gehalt in der Reaktionsmasse von 26 - 28% aufrechtgehalten werden.

Die Biogasproduktivität betrug somit 6,5 Nm³ (tr) pro m³ Reaktor und Tag.

Die Biogasausbeute betrug 144 Nm³ (tr) pro t Input.

### Beispiel 2:

In derselben Fermentationsanlage des 1.Beispiels wurden wöchentlich 5000 kg Bioabfall mit TM = 32% und TOC = 37% TM fermentiert. Das Beschickungsverhältnis Frischmasse zu Reaktionsmasse betrug 1:4, dasjenige der Rückführung 1:2,5. In einer Woche wurden bei einer hydraulischen Verweilzeit von 25 Tagen 1130 kg Biogas produziert. 4600 kg an Reaktionsmasse wurden in der Woche ausgetragen und auf 50% entwässert. Davon wurden ca.20% im entwässerten Zustand zurückgeführt. Der TM-Gehalt in der Reaktionsmasse stellte sich auf 27 - 29% ein.

Die Biogasproduktivität betrug somit 6,0 Nm³ (tr) pro m³ Reaktor und Tag.

Die Biogasausbeute betrug 186 Nm³ pro t Input.

### Beispiel 3:

In einen großtechnischen Reaktor mit 2000 m³ Inhalt (D:H = 1:1,4) wurden wöchentlich 380 t aus Haushalten getrennt gesammelter Bioabfall eingetragen. Der

Bioabfall hatte folgende Eigenschaften: TM=31%, TOC=37% TM. Die täglich ausgetragenen Mengen an Reaktionsmasse und Biogas wurden äquivalent durch frischen Bioabfall ersetzt und im Verhältnis 1:5 mit Reaktionsmasse vermischt in den Reaktor eingetragen. Das diesbezügliche Verhältnis der Rückführungsschlaufe betrug 1:3. Die Reaktionsmischung wurde mit rund 30t Dampf im Mischer auf Temperatur von 55°C gebracht. Nach einer hydraulischen Verweilzeit von 30 Tagen entwickelten sich 85 t Biogas pro Woche. In dieser Zeit wurden 395 t an Reaktionsmasse ausgetragen und auf 50% TM entwässert. Davon wurden etwa 20% der entwässerten Masse wieder in den Reaktor zurückgeführt. In der Reaktionsmasse stellt sich ein TM-Gehalt von 26-28%.

Die Biogasproduktivität betrug somit 5,0 Nm³ (tr) pro m³ Reaktor und Tag.

Die Biogasausbeute betrug 183 Nm³ (tr) pro t Input.

Das erfindungsgemäße Verfahren ist auch besonders geeignet für die Fermentation von organisch abbaubaren Abfällen mit hohem Stickstoffgehalt, weil über die Rückführungsschlaufe die wasserlöslichen Ammoniumverbindungen, die sich in der Wasserphase anreichern, mit dem Preßwasser ausgetragen werden können. Auf diese Weise kann durch die Größe des Anteiles der entwässerten Reaktionsmasse die Konzentration der Ammoniumverbindungen im Fermenter auf ein solches Konzentrationsniveau eingeregelt werden, sodaß keine Hemmung der Abbaureaktion eintritt. Durch Zugabe von Wasser oder stickstoffarmen Abwässern läßt sich die Stickstoffextraktion unterstützen und die erforderliche optimale Feststoffkonzentration in der Reaktionsmasse einhalten.

In gleichartiger Weise kann ein hoher Salzgehalt im Reaktorimput, im besonderen NaCl, durch Erhöhung des Anteiles an entwässerter und zurückgeführter Reaktionsmasse reduziert werden. Dies ist ein weiterer besonderer Vorteil des gegenständlichen Verfahrens, weil dadurch ein bemerkenswert salzarmes Kompostprodukt erzeugt wird. Durch Zugabe von Wasser oder salzarmen Abwässern kann die Feststoffkonzentration auf das technisch optimale Abbauniveau gehalten und im Effekt die Entsalzung beschleunigt werden.

Grundsätzlich kann diese Vorgangsweise für alle störenden Schadstoffe angewendet werden, die in Wasser sehr gut löslich sind.

Die gegenständliche Erfindung wird in keiner Weise auf die vorangegangenen Beschreibungen und Ausführungen beschränkt, im Gegenteil, es sind viele andere Ausführungsvarianten und Reaktionsweisen möglich, welche die kennzeichnenden Merkmale der Erfindung aufweisen und durch die Patentansprüche abgedeckt werden. Im Einzelnen betrifft dies unter anderem die Austragsvorrichtung des Reaktors, die sich nicht auf die zwei Förderschnecken beschränken muß - die Gestaltung der Rückführung von Preßkuchen nicht nur zum Teil über die Beschickungsschlaufe, sondern über eine vollständig getrennte Schlaufe mit eigener Verdrängerpumpe samt Mischvorrichtung - und die Vorrichtung zum Entwässern, bei der an Stelle einer Schneckenpresse auch ein Dekanter oder vorzugsweise eine Kolbenpresse verwendet werden kann.

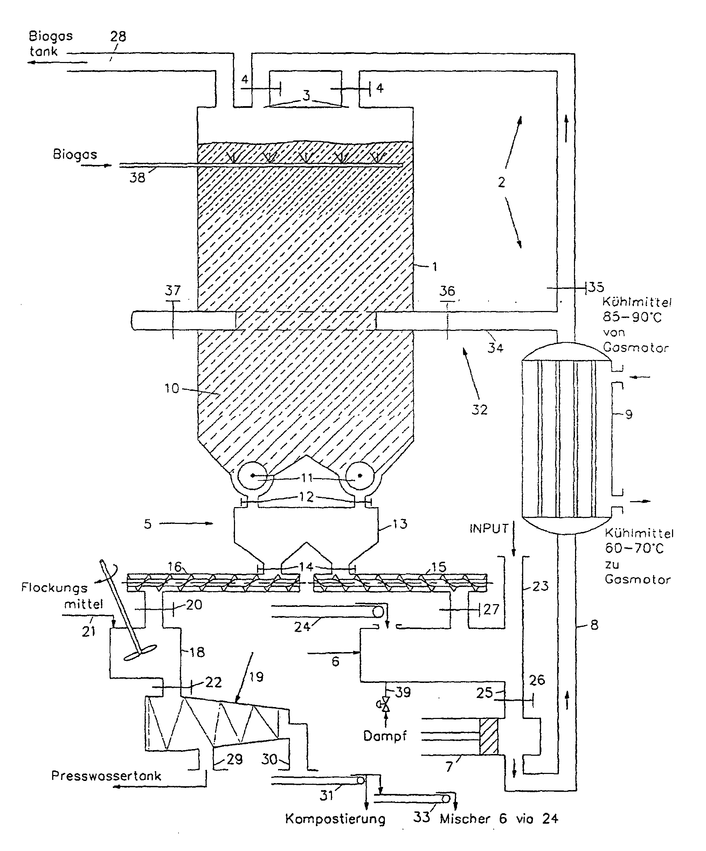

## Patentansprüche

1. Verfahren zum biologischen anaeroben Abbau von organischen Abfällen unter Bildung von Biogas, bei welchem frischer, gegebenenfalls mit in Fermentation befindlichem Material vermischter, Abfall mit einem hohen Trockensubstanzgehalt oben auf das in Fermentation befindliche Material aufgegeben wird, **dadurch gekennzeichnet, daß**, das in Fermentation befindliche, vorzugsweise entwässerte, Material in zwei Materialstromschlaufen rückgeführt wird, wobei ein Teilstrom jener ist, der nach Vermischung mit frischem Material oben auf die Substratschicht aufgegeben wird und der zweite Teilstrom in etwa der halben Höhe der Substratschicht eingeführt wird, wobei der Trockensubstanzgehalt zur Einstellung der Stoffkonzentration in der Reaktionssuspension für den Abbau des organischem Materials geregelt wird.

2. Verfahren nach Anspruch 1, **dadurch gekennzeichnet, daß** frischer Abfall mit in Fermentation befindlichem Material im Verhältnis von 1 : 3 bis 1 : 8, insbesondere 1 : 4 bis 1 : 6, vermischt wird.

3. Verfahren nach Anspruch 1 oder 2, **dadurch gekennzeichnet, daß** zur Nachimpfung in Fermentation befindliches Material im zweiten Strom im Kreislauf gerührt wird, u.zw. bezogen auf frischen Abfall im Verhältnis von 4 : 1 bis 1 : 1, insbesondere von 3 : 1 bis 2 : 1.

4. Verfahren nach einem der Ansprüche 1 bis 3, **dadurch gekennzeichnet, daß** maximal die Hälfte, vorzugsweise ein Drittel bis ein Viertel des gesamten, in Fermentation befindlichen Materials im Kreislauf rückgeführt wird.

5. Verfahren nach einem der Ansprüche 1 bis 4, **dadurch gekennzeichnet, daß** der pH-Wert des frischen Abfalls zwischen 3 und 5 eingestellt wird.

6. Verfahren nach einem der Ansprüche 1 bis 5, **dadurch gekennzeichnet, daß** die Feststoffkonzentration des für das Umwälzen abgezogenen, in Fermentation befindlichen Materials zwischen 20 und 50 Gew.-%, vorzugsweise zwischen 25 und 40 Gew.-% liegt.

7. Verfahren nach Anspruch 6, **dadurch gekennzeichnet, daß** die Feststoffkonzentration durch Entwässern eines Teiles des umgewälzten Gutes und Wiedervereinigen des entwässerten Teiles mit dem Rest des umgewälzten Gutes eingestellt wird.

8. Verfahren nach Anspruch 6, **dadurch gekennzeichnet, daß** zum Einstellen der Feststoffkonzentration, Wasser, Abwässer, Dünnschlämme oder Gülle dem umgewälzten Gut zugesetzt werden.

9. Verfahren nach einem der Ansprüche 1 bis 8, **dadurch gekennzeichnet, daß** zum Entfernen von Schadstoffen das in Fermentation befindliche Material von der Hauptmenge abgezogen, entwässert, vorzugsweise gepreßt, und die abgeführte Flüssigkeitsmenge durch schadstoffarme Flüssigkeit, z.B. Wasser, Abwässer, Dünnschlämme oder Gülle vor dem Rückführen in die Hauptmenge ersetzt wird.

10. Verfahren nach einem der Ansprüche 1 bis 9, **dadurch gekennzeichnet, daß** der Ammoniumstickstoff in dem in Fermentation befindlichen Gut auf maximal 4 g/kg gehalten wird, wobei gegebenenfalls dem Gut Nährstoffe für die anaeroben Mikroorganismen beigemengt werden.

11. Vorrichtung zur Durchführung des Verfahrens nach einem der Ansprüche 1 bis 10, welche ein Reaktionsgefäß mit Abzugsleitung für Biogas, eine Beschickungseinrichtung, eine Austrageinrichtung, eine Entwässerungsvorrichtung und eine Mischvorrichtung aufweist, **dadurch gekennzeichnet, daß** die Beschickungseinrichtung als Beschickungsschlaufe (2) ausgebildet ist, die oberhalb der Substratschicht (10) in das Reaktionsgefäß (1) einmündet, und daß zusätzlich eine Umwälzschlaufe (32) vorgesehen ist, die im Bereich von 25 - 70 %, insbesondere 30 - 50 %, der Substratschichthöhe in das Reaktionsgefäß (1) einmündet.

12. Vorrichtung nach Anspruch 11, **dadurch gekennzeichnet, daß** sowohl die Beschickungsschlaufe (2) als auch die Umwälzschlaufe (32) von der Mischvorrichtung (6) ausgehen.

13. Vorrichtung nach Anspruch 11 oder 12, **dadurch gekennzeichnet, daß** das Verhältnis von Durchmesser zur Höhe des Reaktionsgefäßes (1) zwischen 1 : 1,3 bis 1 : 4 beträgt.

14. Vorrichtung nach einem der Ansprüche 11 bis 13, **dadurch gekennzeichnet, daß** die Austrageinrichtung (5) durch eine oder mehrere Förderschnecken (11) gebildet ist, wobei der Boden des Reaktionsgefäßes (1) zu der bzw. den Förderschnecken (11) hin geneigt verläuft.

15. Vorrichtung nach Anspruch 14, **dadurch gekennzeichnet, daß** bei Vorhandensein von zwei Förderschnecken (11) der Boden des Reaktionsgefäßes (1) hosenförmig ausgebildet ist.

16. Vorrichtung nach Anspruch 14 oder 15, **dadurch gekennzeichnet, daß** die Neigung der Bodenwandung mindestens 20° beträgt.

17. Vorrichtung nach einem der Ansprüche 14 bis 16, **dadurch gekennzeichnet, daß** den Förderschnecken (11) ein Zwischenbehälter (13) nachgeschaltet ist, der einlaufseitig und auslaufseitig mit Absperrschiebern (12, 14) versehen ist.

18. Vorrichtung nach Anspruch 17, **dadurch gekennzeichnet, daß** dem Zwischenbehälter (13) die Entwässerungseinrichtung (19) nachgeschaltet ist, die nach dem Preßprinzip, gegebenenfalls unter Einsatz eines Flockungsmittels, arbeitet.

19. Vorrichtung nach Anspruch 18, **dadurch gekennzeichnet, daß** der Entwässerungseinrichtung (19) ein Preßwassertank nachgeschaltet ist, der mit der Mischvorrichtung (6) in Verbindung steht, welche ihrerseits vom Zwischenbehälter (13) beschickbar ist.

20. Vorrichtung nach Anspruch 19, **dadurch gekennzeichnet,daß** der Auslaß (30) der Entwässerungseinrichtung (19) über Förderorgane (31, 33, 24) mit der Mischvorrichtung (6) verbindbar ist.

21. Vorrichtung nach einem der Ansprüche 11 bis 20, **dadurch gekennzeichnet, daß** im gemeinsam verlaufenden Teil der Beschickungsschlaufe (2) und der Umwälzschlaufe (32) eine direkt oder indirekt wirkende Erwärmungseinrichtung für das strömende Gut vorgesehen ist.

22. Vorrichtung nach Anspruch 21, **dadurch gekennzeichnet, daß** als Erwärmungseinrichtung ein Wärmetauscher (9) vorgesehen ist, der mit dem Kühlwasser eines mit dem Biogas aus dem Reaktionsgefäß (1) betriebenen Motors beschickt ist.

## Claims

1. A method of biologically, and anaerobically decomposing organic waste material whilst producing biogas, in which fresh waste material, optionally mixed with fermenting material, with a high dry substance content is supplied onto the top of the fermenting material, **characterised in that** the fermenting, preferably dewatered, material is recirculated in two material flow loops, whereby one flow is that which, after mixing with fresh material, is supplied onto the top of the substrate layer and the second flow is introduced about half way up the substrate layer, whereby the dry substance content is controlled to adjust the material concentration in the reaction suspension for the decomposition of the organic material.

2. A method as claimed in claim 1, **characterised in that** fresh waste material is mixed with fermenting material in the ratio of 1:3 to 1:8, particularly 1:4 to 1:6.

3. A method as claimed in claim 1 or 2, **characterised in that** fermenting material is conducted in a circuit in the second flow, for the purpose of post-inoculation, in the ratio of 4:1 to 1:1, particularly 3:1 to 2:1, with respect to fresh waste material.

4. A method as claimed in one of claims 1-3, **characterised in that** at most one half, preferably one third to one quarter, of the total fermenting material is recirculated in the circuit.

5. A method as claimed in one of claims 1-4, **characterised in that** the pH value of the fresh waste material is set to be between three and five.

6. A method as claimed in one of claims 1-5, **characterised in that** the solid material concentration of the fermenting material withdrawn for circulation is between 20% and 50% by wt, preferably between 25% and 40% by wt.

7. A method as claimed in claim 6, **characterised in that** the solid material concentration is adjusted by dewatering a proportion of the circulated material and recombining the dewatered proportion with the remainder of the circulated material.

8. A method as claimed in claim 6, **characterised in that** water, waste water, thin slurry or semi-liquid manure is added to the circulated material in order to adjust the solid concentration.

9. A method as claimed in one of claims 1-8, **characterised in that**, in order to remove contaminants, the fermenting material is withdrawn from the main volume, dewatered and preferably pressed and the volume of liquid which was drained off is replaced by low-contaminant liquid, e.g. water, waste water, thin slurry or semi-liquid manure before returning it to the main volume.

10. A method as claimed in one of claims 1-9, **characterised in that** the ammonia-nitrogen in the fermenting material is maintained at a maximum of 4g/kg, whereby nutrients are optionally added to the material for the anaerobic microorganisms.

11. An apparatus for carrying out the method as claimed in one of claims 1-10, which includes a reaction vessel with a discharge conduit for biogas, a charging device, a discharging device, a dewatering device and a mixing device, **characterised in that** the charging device is constructed in the form of a charging loop (2), which communicates with the reaction vessel (1) above the substrate layer (10) and that additionally a circulation loop (32) is provided, which communicates with the reaction vessel (1) at within the range of 25%-70%, particularly 30%-50%, of the height of the substrate layer.

12. Apparatus as claimed in claim 11, **characterised in that** both the charging loop (2) and the circulation loop (32) start from the mixing device (6).

13. Apparatus as claimed in claims 11 or 12, **characterised in that** the ratio of the diameter to height of the reaction vessel (1) is between 1:1.3 to 1:4.

14. Apparatus as claimed in one of claims 11-13, **characterised in that** the discharge device (5) is constituted by one or more conveyor screws (11), the base of the reaction vessel (1) extending at an inclination to the conveyor screw(s) (11).

15. Apparatus as claimed in claim 14, **characterised in that** when two conveyor screws (11) are present, the base of the reaction vessel (1) is shaped in the manner of a pair of trousers.

16. Apparatus as claimed in claims 14 or 15, **characterised in that** the inclination of the base wall is at least 20°.

17. Apparatus as claimed in claim one of claims 14-16, **characterised in that** connected downstream of the conveyor screws (11) there is an intermediate container (13), which is provided on its inlet side and its outlet side with gate valves (12, 14).

18. Apparatus as claimed in claim 17, **characterised in that** connected downstream of the intermediate container (13) is the dewatering device (19), which operates on the pressing principal, optionally with the use of a flocculant.

19. Apparatus as claimed in claim 18, **characterised in that** connected after the dewatering device (19) there is a pressurised water tank, which is in communication with the mixing device (6), which for its part may be charged from the intermediate container (13).

20. Apparatus as claimed in claim 19, **characterised in that** the outlet (30) of the dewatering device (19) is connectable by means of conveying elements (31, 33, 24) to the mixing device (6).

21. Apparatus claimed in one of claims 11-20, **characterised in that** a directly or indirectly operating heating device for the flowing material is provided in the common portion of the charging loop (2) and the circulation loop (32).

22. Apparatus claimed in claim 21, **characterised in that** a heat exchanger (9) is provided as the heating device, which is fed with the cooling water from a motor operated with the biogas from the reaction vessel (1).

## Revendications

1. Procédé pour la dégradation biologique anaérobie de déchets organiques avec production de biogaz, dans lequel on dépose un déchet frais avec une teneur élevée en substance sèche, le cas échéant mélangé avec une matière en cours de fermentation, au-dessus de la matière en cours de fermentation, **caractérisé en ce que** la matière, de préférence égouttée, en cours de fermentation est remise en circulation dans deux boucles du flux de la matière, moyennant quoi une première fraction de flux de cette matière est celle qui est déposée, après mélange avec de la matière fraîche, au dessus d'une couche de substrat et la seconde fraction de flux est introduite à environ mi-hauteur dans la couche de substrat, moyennant quoi la teneur en matière sèche est régulée pour l'ajustement de la concentration des matières solides dans la suspension de réaction destinée à la dégradation de la matière organique.

2. Procédé selon la revendication 1, **caractérisé en ce que** le déchet frais est mélangé avec la matière en cours de fermentation dans une proportion comprise entre 1:3 et 1:8, en particulier, entre 1:4 et 1:6.

3. Procédé selon la revendication 1 ou 2, **caractérisé en ce que**, pour la germination ultérieure, la matière en cours de fermentation est mise en circulation dans le second flux, et notamment dans une proportion, par rapport au déchet frais, comprise entre 4:1 et 1:1, en particulier, entre 3:1 et 2:1.

4. Procédé selon l'une quelconque des revendications 1 à 3, **caractérisé en ce que**, au maximum la moitié, de préférence un tiers à un quart de la totalité de la matière en cours de fermentation, est remise en circulation.

5. Procédé selon l'une quelconque des revendications 1 à 4, **caractérisé en ce que** la valeur pH de la matière fraîche est ajustée entre 3 et 5.

6. Procédé selon l'une quelconque des revendications 1 à 5, **caractérisé en ce que** la concentration en matières solides de la matière en cours de fermentation, extraite pour le recyclage, représente une masse pondérale comprise entre 20 et 50%, de préférence entre 25 et 40%.

7. Procédé selon la revendication 6, **caractérisé en ce que** la concentration en matières solides est ajustée, au moyen de l'égouttage d'une partie de la masse recyclée et de la recombinaison de la partie égouttée avec le reste de la masse recyclée.

8. Procédé selon la revendication 6, **caractérisé en ce que**, pour ajuster la concentration en matières solides, on ajoute de l'eau, des eaux usées, des boues clarifiées ou du lisier à la masse recyclée.

9. Procédé selon l'une quelconque des revendications 1 à 8, **caractérisé en ce que**, pour éliminer les matières polluantes, la matière en cours de fermentation est extraite de la quantité principale, est égouttée, de préférence, est pressée et la quantité liquide retirée est remplacée par un liquide faiblement polluant, par exemple, de l'eau, des eaux usées, des boues clarifiées ou du lisier avant d'être réintroduite dans la quantité principale.

10. Procédé selon l'une quelconque des revendications 1 à 9, **caractérisé en ce que** de l'azote ammoniacal est maintenu dans la masse en cours de fermentation au maximum à 4 g/kg, moyennant quoi, le cas échéant, des nutriants destinés aux microorganismes anaérobiques sont amalgamés dans la masse.

11. Dispositif pour mettre en oeuvre le procédé selon l'une quelconque des revendications 1 à 10, lequel présente une cuve de réaction comportant une conduite d'évacuation pour le biogaz, un système de chargement, un système de vidage, un dispositif d'égouttage et un dispositif mélangeur, **caractérisé en ce que** le système de chargement est conçu comme une boucle de chargement (2), qui débouche dans la cuve de réaction au-dessus de la couche de substrat (10), et **en ce qu'**il est prévu, en plus, une boucle de recyclage (32) qui débouche dans la cuve de réaction (1) dans une région correspondant entre 25 et 70%, en particulier entre 30 et 50% à 1a hauteur de la couche de substrat.

12. Dispositif selon la revendication 11, **caractérisé en ce que** tant la boucle de chargement (2) que la boucle de recyclage (32) sortent du dispositif mélangeur (6).

13. Dispositif selon la revendication 11 ou 12, **caractérisé en ce que** le rapport du diamètre par rapport à la hauteur de la cuve de réaction (1) est compris entre 1:1,3 et 1:4.

14. Dispositif selon l'une quelconque des revendications 11 à 13, **caractérisé en ce que** le système de vidage (5) est formé par une ou plusieurs vis transporteuses (11), moyennant quoi le fond de la cuve de réaction (1) s'étend selon un plan incliné respectivement vers les vis transporteuses (11).

15. Dispositif selon la revendication 14, **caractérisé en ce que**, dans le cas où il y a deux vis transporteuses (11), le fond de la cuve de réaction (1) est conçu dans une forme d'un tuyau divisé.

16. Dispositif selon la revendication 14 ou 15, **caractérisé en ce que** l'inclinaison de la paroi de fond est d'au moins 20°.

17. Dispositif selon l'une quelconque des revendications 14 à 16, **caractérisé en ce qu'**il est monté, en amont des vis transporteuses (11), une cuve intermédiaire (13) qui est pourvue, du côté de l'écoulement entrant et du côté de l'écoulement sortant, de vannes d'arrêt (12, 14).

18. Dispositif selon la revendication 17, **caractérisé** en ce en ce qu'il est monté, en amont de la cuve intermédiaire (13), un système d'égouttage (19) qui fonctionne selon le principe de pressage, le cas échéant, avec utilisation d'un floculant.

19. Dispositif selon la revendication 18, **caractérisé en ce qu'**il est monté, en amont du système d'égouttage (19,) un réservoir d'eau de pressage qui est relié au dispositif mélangeur (6), lequel peut, de son côté, être chargé par la cuve intermédiaire (13).

20. Dispositif selon la revendication 19, **caractérisé en ce que** l'évacuation (30) du système d'égouttage (19) peut être reliée au dispositif mélangeur au moyen d'organes de transport (31, 33, 24).

21. Dispositif selon l'une quelconque des revendications 11 à 20, **caractérisé en ce que**, dans une partie s'étendant de façon commune entre la boucle de chargement (2) et la boucle de circulation (32), il est prévu un système d'échauffement à action directe ou indirecte de la masse en écoulement.

22. Dispositif selon la revendication 21, **caractérisé en ce qu'**il est prévu, en tant que système d'échauffement, un échangeur de chaleur (9) qui est chargé avec l'eau de refroidissement d'un moteur actionné avec le biogaz provenant de la cuve de réaction (1).
